Europäisches Patentamt

(19) European Patent Office

Office européen des brevets

(11) **EP 0 597 029 B1**

(12) **EUROPEAN PATENT SPECIFICATION**

(45) Date of publication and mention
of the grant of the patent:
**15.01.1997 Bulletin 1997/03**

(21) Application number: **92917845.7**

(22) Date of filing: **24.07.1992**

(51) Int. Cl.$^6$: **A61K 9/107**

(86) International application number:
**PCT/US92/06181**

(87) International publication number:
**WO 93/02665 (18.02.1993 Gazette 1993/05)**

(54) **W/O MICROEMULSIONS**

W/O MIKROEMULSIONEN

MICROEMULSION HUILEUSE

(84) Designated Contracting States:
**AT BE CH DE DK ES FR GB GR IT LI LU MC NL SE**

(30) Priority: **26.07.1991 US 736352**

(43) Date of publication of application:
**18.05.1994 Bulletin 1994/20**

(73) Proprietor: **SMITHKLINE BEECHAM CORPORATION**
**Philadelphia Pennsylvania 19101 (US)**

(72) Inventor: **CONSTANTINIDES, Panayiotis Pericleous**
**Devon, PA 19333 (US)**

(74) Representative: **Thompson, Clive Beresford**
**SmithKline Beecham plc**
**Corporate Intellectual Property,**
**Two New Horizons Court**
**Brentford, Middlesex TW8 9EP (GB)**

(56) References cited:
**EP-A- 0 278 660        FR-A- 1 603 312**
**US-A- 4 840 660**

EP 0 597 029 B1

## Description

This invention relates to pharmaceutically acceptable water-in-oil (w/o) self-emulsifying microemulsions containing therapeutic agents, processes for their preparation and their use.

Microemulsions can be defined in general as thermodynamically stable, isotropically clear dispersions of two immiscible liquids stabilized by interfacial films of surface-active molecules. The formation of microemulsions usually involves a combination of three to five components, namely, an oil, water, a surfactant, a cosurfactant and an electrolyte. The tendency towards a water-in-oil (w/o) or an oil-in-water (o/w) microemulsion is dependent on the properties of the oil and the surfactant. Surfactants are conveniently classified on an empirical scale known as the hydrophilic-lipophilic balance (HLB) which runs from 1 to 20. In general, (w/o) microemulsions are formed using surfactants (or emulsifiers) which have an HLB value in the range of about 3 to 6 whilst (o/w) microemulsions are formed using surfactants which have an HLB value in the range of about 8 to 18. It has long been recognized that low interfacial tension contributes to the thermodynamic stability of microemulsions. To achieve this, the surfactant should preferably exhibit low solubility in both the oil and water phases, and be preferentially absorbed at the water-oil interface with concomitant lowering of interfacial tension. When interfacial tension is less than $2 \times 10^{-7}$ N/cm ($2 \times 10^{-2}$ dyn/cm), a stable microemulsion can form. General reviews of microemulsions are provided by Bhargava $et\ al.$, Pharm. Tech., 46-53, March 1987 and Kahlweit, Science, **240**, 617-621, 1988.

Microemulsions are typically substantially non-opaque, i.e. are transparent or opalescent when viewed by optical microscopic means. In the undisturbed state, they are optically isotropic (non-birefringent) when examined under polarized light. The dispersed phase typically comprises particles or droplets which are normally between 5 and 200 nm in size and this gives rise to their optical transparency. These particles may be spherical although other structures are feasible.

The role of the cosurfactant, usually a short-chain alcohol, is to increase the interfacial fluidity by penetrating the surfactant film and consequently creating a disordered film due to the void space among surfactant molecules. The use of a cosurfactant in microemulsions is however optional and alcohol-free self-emulsifying emulsions and microemulsions have been described in the literature (see for instance, Pouton et al., Int,. Journal of Pharmaceutics, **27**, 335-348, 1985 and Osborne $et\ al.$, J. Disp. Sci. Tech., **9**, 415-423, 1988).

There are many advantages to the use of a microemulsion over a conventional emulsion (or macroemulsion) for drug transport (delivery). Microemulsions form spontaneously, without the need for a high input of energy and are therefore easy to prepare and scale up for commercial applications; they have thermodynamic stability due to their small particle size and therefore have a long shelf life; they have an isotropically clear appearance so that they may be monitored by spectroscopic means; they have a relatively low viscosity and are therefore easy to transport and mix; they have a large interfacial area which accelerates surface reactions; they have a low interfacial tension which permits flexible and high penetrating power and, lastly, they offer the possibility of improved drug solubilization and protection against enzymatic hydrolysis. In addition, microemulsions may undergo phase inversion upon addition of an excess of the dispersed phase or in response to a temperature change and this is a property of these systems that can affect drug release from microemulsions both $in\ vitro$ and $in\ vivo$. The reasons for this improved drug delivery are not however well understood.

Lipid-based microemulsions have already been proposed to enhance the bioavailability of different drugs, including peptides. Thus, GB 2 222 770-A (Sandoz Ltd) describes microemulsions and corresponding microemulsion "pre-concentrates" for use with the highly hydrophobic cyclosporin peptides. Thus, a suitable pre-concentrate comprises 1,2-propylene glycol as the hydrophilic phase, a caprylic-capric acid triglyceride as the lipophilic component and a mixture of a polyoxyethylene glycolated hydrogenated castor oil and glycerin monooleate (ratio 11:1) as the surfactant-cosurfactant. Such formulations may then be diluted with water but to give an oil-in-water rather than a water-in-oil microemulsion.

In addition, GB 2 098 865-A (Sandoz Ltd) describes topical compositions in the form of microemulsions comprising a water-immiscible organic solvent, an emulsifier, a co-emulsifier, water and a (non-peptide) therapeutic agent. These formulations are said to have improved skin penetrating properties. Suitable organic solvents include ($C_{10-22}$)-fatty acid esters of ($C_{3-18}$)-alcohols such as hexyl laurate, ($C_{12-32}$)-hydrocarbons such as squalene and mono- or diesters of glycerol with a ($C_{6-22}$)-carboxylic acid such as glyceryl caprylate (which may also act as a co-emulsifier). There is however no mention of using a long chain-fatty acid triglyceride as the oil.

Furthermore, US 4 712 239 (Muller $et\ al.$) describes multi-component systems for pharmaceutical use comprising an oil, a nonionic surfactant with an HLB value above 8 and a co-surfactant which is a partial ether or ester of a polyhydroxyl alcohol and a ($C_{6-22}$) fatty alcohol or acid, which components form a "single phase" on mixing. The special properties of the system are attributed to the particular blend of surfactant and co-surfactant selected. An aqueous phase is an optional extra and the therapeutic agent may be lipophilic or hydrophilic. Such systems are said to give enhanced transdermal delivery characteristics. Although examples of formulations are provided comprising medium-chain fatty acid triglycerides and medium-chain fatty acid mono- and di-glycerides, there are no corresponding examples using long-chain analogues thereof.

Finally, WO 88/00059 (Engström $et\ al.$ and the corresponding paper, J. Dispersion Sci. Technol., 11, 479, 1990) discloses controlled release compositions for biologically active materials comprising an "L2-phase" and containing an unsaturated ($C_{16-22}$)-, preferably $C_{18}$-,fatty acid monoglyceride and an unsaturated ($C_{16-22}$)-, preferably $C_{18}$-, fatty acid triglyceride, in a ratio of from 1:1 to 3:1, and a polar liquid such as water. There is however no mention of the additional inclusion of a high HLB surfactant. The existence of an L2 phase had previously been described, in the context of medium-chain fatty acid components, for a water-monocaprylin-tricaprylin system by Friberg *et al.*, J. Amer. Oil Chem. Soc., **47**, 149, 1970. Again, there is no mention of the additional inclusion of a high HLB surfactant.

EP-A-278660 and FR-A-1603312 disclose microemulsions for pharmaceutical use.

We have now surprisingly found that further improved drug delivery characteristics may be obtained using (w/o) microemulsions having lipophilic phases based on a long-chain fatty acid triglyceride and a low HLB surfactant, in combination with a high HLB surfactant and an aqueous based hydrophilic phase.

Accordingly, the present invention provides a pharmaceutically acceptable, stable, self-emulsifying water-in-oil (w/o) microemulsion comprising:

(a) a lipophilic phase having a long-chain fatty acid triglyceride and a low HLB surfactant;
(b) a high HLB surfactant;
(c) an aqueous hydrophilic phase; and
(d) a water-soluble therapeutic agent.

The accompanying drawings contain the following figures:

Figure 1 illustrates a pseudo-ternary phase diagram reading of a microemulsion system containing an oil and a low HLB surfactant in a fixed ratio X, a high HLB surfactant and an aqueous phase;

Figure 2 illustrates a pseudo-ternary phase diagram of the microemulsion system soybean oil/MYVEROL 18-99 as the oil/low HLB surfactant in a ratio of 3 to 1, Tween 80 as the high HLB surfactant and water as the aqueous phase;

Figure 3 illustrates a pseudo-ternary phase diagram of the microemulsion system soybean oil/MYVEROL 18-99 as the oil/low HLB surfactant in a ratio of 3 to 1, Tween 80 as the high HLB surfactant and saline as the aqueous phase;

Figure 4 illustrates a pseudo-ternary phase diagram of the microemulsion system soybean oil/ARLACEL 186 as the oil/low HLB surfactant in a ratio of 3 to 1, Tween 80 as the high HLB surfactant and water as the aqueous phase;

Figure 5 illustrates a pseudo-ternary phase diagram of the microemulsion system soybean oil/sorbitan sesquioleate as the oil/low HLB surfactant in a ratio of 3 to 1, Tween 80 as the high HLB surfactant and water as the aqueous phase;

Figure 6 illustrates a pseudo-ternary phase diagram of the microemulsion system soybean oil/sorbitan monooleate as the oil/low HLB surfactant in a ratio of 3 to 1, Tween 80 as the high HLB surfactant and water as the aqueous phase; and

Figure 7 illustrates a pseudo-ternary phase diagram of the microemulsion system soybean oil/sorbitan monooleate as the oil/low HLB surfactant in a ratio of 4 to 1, Tween 80 as the high HLB surfactant and water as the aqueous phase.

Suitable long-chain fatty acid triglycerides for use in the present invention may be of natural, semi-synthetic or synthetic origin and may include blends of different long-chain fatty acid triglycerides. The term "long-chain fatty acid" as used herein refers to a fatty acid which may be saturated, mono-unsaturated or poly-unsaturated, having from 12 to 22, preferably 14 to 18, carbon atoms which may be branched or unbranched, preferably unbranched, and which may be optionally substituted. Certain neutral, natural or hydrogenated plant, vegetable and fish oils such as shark oil, olive oil, sesame oil, peanut oil, natural and hydrogenated castor oils, safflower oil, sunflower oil and soybean oil provide convenient sources of long-chain fatty acid triglycerides. Soybean oil consists of oleic acid (25%), linoleic acid (54%), linolenic acid (6%), palmitic acid (11%) and stearic acid (4%) triglycerides whilst safflower oil consists of oleic acid (13%), linoleic acid (76%), stearic acid (4%) and palmitic acid (5%) triglycerides. Suitably in such long-chain fatty acid triglycerides, the major fatty acid components are $C_{18}$-saturated, monounsaturated or polyunsaturated fatty acids, preferably $C_{18}$-monounsaturated or polyunsaturated fatty acids.

Suitable low HLB surfactants for use in the present invention include long-chain fatty acid monoglycerides, optionally comprising up to 10% (w/w) of a long-chain fatty acid diglyceride and/or a small amount by weight of a free long-chain fatty acid. The mono- and di-glycerides may each include blends of different long-chain fatty acid mono- and diglycerides. Suitable long-chain fatty acid monoglycerides include glycerol monooleate, glycerol monopalmitate and glycerol monostearate. Suitable commercially available examples of such include the products available under the trade names MYVEROL, such as MYVEROL 18-99, MYVATEX and MYVAPLEX, respectively, from Eastman Kodak Chemicals, Rochester, New York. A further useful long-chain fatty acid monoglyceride-containing product is ARLACEL 186 (available from ICI Americas Inc.) which includes, in addition to glycerol monooleate, propylene glycol (10%). The main fatty acids of MYVEROL 18-99 are oleic acid (61%), linoleic acid (21%), linolenic acid (9%) and palmitic acid (4%). Suitably in such long-chain monoglycerides, the major fatty acid component is a $C_{18}$-saturated, monounsaturated or poly-unsaturated fatty acid, preferably a $C_{18}$-monounsaturated or polyunsaturated fatty acid. In addition, diacetylated versions of the monoglycerides such as the product available under the trade name MYVATEX SMG are also useful. Further suitable low HLB surfactants for use in the present invention include sorbitan long-chain fatty acid esters such as sorbitan monooleate, available commercially under the trade names SPAN 80 and ARLACEL 80 and sorbitan sesquioleate, available commercially under the trade names SPAN 83 and ARLACEL 83. Suitably the low HLB surfactant will have an HLB value in the range of about 2.5 to 6. The HLB values of the products MYVEROL 18-99, ARLACEL 80, ARLACEL 83 and ARLACEL 186 are respectively 3.7, 4.3, 3.7 and 2.8.

Suitable combinations of long-chain fatty acid triglycerides and low HLB surfactants include soybean oil or safflower oil and glycerol monooleate, sorbitan monooleate or sorbitan sesquioleate.

The use in a self-emulsifying (w/o) microemulsion according to the present invention of a low HLB surfactant which is a long-chain fatty acid monoglyceride optionally containing a long-chain fatty acid diglyceride or which is a sorbitan long-chain fatty acid ester as hereinbefore defined and which is a component of the lipophilic phase provides for reduced droplet size and this is believed to aid in the absorption of the therapeutic agent.

Suitable high HLB surfactants for use in the present invention include non-ionic surfactants such as (a) polyoxyethylene fatty acid esters, for example polyoxyethylene stearic acid esters of the type available under the trade name MYRJ (ICI Americas, Inc.), for instance the product MYRJ 52 (a polyoxyethylene 40 stearate); (b) polyoxyethylene-sorbitan fatty acid esters (polysorbates), for example the mono- and tri-lauryl, palmityl, stearyl and oleyl esters, for instance the polyoxyethylene sorbitan monooleates available under the trade name of TWEEN (ICI Americas Inc.), such as TWEEN 20, 21, 40, 60, 61, 65, 80, 81 and 85, of which class TWEEN 80 is especially preferred; (c) polyoxyethylene glycol long-chain alkyl ethers, such as polyoxyethylated glycol lauryl ether; and (d) polyoxyethylene glycol long-chain alkyl esters, such as PEG-monostearate.

For use herein, the high HLB surfactant preferably has an HLB value in the range of 13 to 20. The products MYRJ 52 and TWEEN 80 have HLB values of 16.9 and 15.0 respectively.

Suitably, the blend of low and high HLB surfactants will have an HLB value in the range of from about 5 to about 8.

As used herein, the term "therapeutic agent" (hereinafter referred to as "drug") refers to any compound which has biological activity, is soluble in the hydrophilic phase and has an HLB value of at least that of the high HLB surfactant used in the formulation, to ensure that the drug is preferentially dissolved in the hydrophilic rather than the lipophilic phase. Suitable drugs include both peptides and non-peptides. Suitable peptides include not only small peptides but also larger peptides or polypeptides and proteins. Suitable such peptides preferrably have a molecular weight from about 100 to 10,000, more preferably from about 100 to about 6,000. Especially preferred peptides have from 2 to 35 amino acid moities. Higher molecular weight polypeptides, with a molecular weight above 10,000 and up to about 50,000, may also be accomodated in microemulsions of the present invention.

Suitable small peptides have from about 2 to about 10, more preferably from about 2 to about 6 amino acid moieties. Preferred small peptides include the fibrinogen receptor antagonists (RGD containing peptides) which are tetrapeptides with an average molecular weight of about 600. These peptide antagonists are highly potent platelet aggregation inhibitors at plasma levels as low as 1 pmol/ml. Preferred fibrinogen antagonists include the peptide cyclo(S,S)-$N^a$-acetyl-Cys-($N^a$-methyl)Arg-Gly-Asp-Pen-$NH_2$ (Ali et al., EP 0 341 915) and the peptide cyclo(S,S)-(2-mercapto)benzoyl-($N^a$-methyl)Arg-Gly-Asp-(2-mercapto)phenylamide (EP 0 423 212). Other fibrinogen antagonists useful in the present invention are those peptides disclosed by Pierschbacher et al., WO 89/05150 (US/88/04403); Marguerie, EP 0 275 748; Adams et al., U.S. 4,857,508; Zimmerman et al., U.S. 4,683,291; Nutt et al., EP 0 410 537, EP 0 410 539, EP 0 410 540, EP 0 410 541, EP 0 410 767, EP 0 410 833, EP 0 422 937 and EP 0 422 938; Ali et al., EP 0 372 486; Ohba et al., WO 90/02751 (PCT/JP89/00926); Klein et al., U.S. 4,952,562; Scarborough et al., WO 90/15620 (PCT/US90/03417); Ali et al., PCT/US90/06514 and PCT/US92/00999; the peptide-like compounds disclosed by Ali et al., EP 0 381 033 and EP 0 384 362; and the RGD peptide cyclo-$N^a$-acetyl-Cys-Asn-Dtc-Amf-Gly-Asp-Cys-OH (in which Dtc is 4,4'-dimethylthiazolidine-5-carboxylic acid and Amf is 4-aminomethylphenylalanine).

The RGD peptide may be usefully included in the microemulsion formulation in an amount up to about 300mg/g of the hydrophilic phase or from 0.1 to 10 mg/g of the formulation.

Other peptides useful in the present invention include, but are not limited to, other RGD containing peptides such as those disclosed by Momany, U.S. 4,411,890 and U.S. 4,410,513; Bowers et al., U.S. 4,880,778, U.S. 4,880,777, U.S. 4,839,344; and WO 89/10933 (PCT/US89/01829); the peptide Ala-His-D-Nal-Ala-Trp-D-Phe-Lys-$NH_2$ (in which Nal represents β-naphthylalanine) and the peptides disclosed by Momany, U.S. 4,228,158, U.S. 4,228,157, U.S. 4,228,156, U.S. 4,228,155, U.S. 4,226,857, U.S. 4,224,316, U.S. 4,223,021, U.S. 4,223,020, U.S. 4,223,019 and U.S. 4,410,512.

Other suitable peptides include hexapeptides such as the growth hormone releasing peptide (GHRP) His-D-Trp-Ala-Trp-D-Phe-Lys-$NH_2$, (Momany, US 4,411,890). This may be usefully included in microemulsions at up to 150mg/g of the hydrophilic phase and from 0.1 to 5mg/g of the formulation.

Suitable polypeptides and proteins for use in microemulsions of the present invention include insulin, calcitonin, elcatonin, calcitonin-gene related peptide and porcine somatostatin as well as analogs and homologs thereof. Other suitable larger polypeptides include those disclosed by Pierschbacher et al., U.S. 4,589,881 (>30 residues); Bittle et al.,

U.S. 4,544,500 (20-30 residues); and Dimarchi *et al.*, EP 0 204 480 (>34 residues).

Other type of compounds useful in the present invention include analogs or homologs of LHRH which display potent LH releasing activity or inhibit the activity of LHRH; analogs or homologs of HP5 which possesses hematopoetic activity; analogs or homologs of endothelin which possess hypotensive activity; analogs or homologs of enkephalin which have antinociceptive activity; analogs or homologs of chlorecystokinin; analogs or homologs of atrial natriuretic factor; peptidergic antineoplastic agents; analogs or homologs of gastrin releasing peptide; analogs or homologs of somatostatin; gastrin antagonists; bradykinin antagonists; neurotensin antagonists; bombesin antagonists; oxytocin agonists and antagonists; vasopressin agonists and antagonists; hirudin abalogs and homologs; abalogs and homologs of the cytoprotective peptide-cyclolinopeptide; alpha MSH analogs; analogs, and homologs of MSH releasing factor (Pro-Leu-Gly-NH$_2$); peptides which inhibit collagenase; peptides which inhibit elastase, peptides which inhibit renin; peptides which inhibit HIV protease; peptides which inhibit angiotensin converting enzyme; peptides which inhibit chymases and tryptases and peptides which inhibit blood coagulation enyzmes.

Other suitable drugs include non-peptide therapeutic agents such as antibotics, antimicrobial agents, antineoplastic agents, cardiovascular and renal agents, antiinflammatory, immunosuppressive and immunostimulatory agents and CNS agents.

Preferably, the drug is a peptide such as a fibrinogen receptor antagonist peptide (an RGD peptide), GHRP (His-D-Trp-Ala-Trp-D-Phe-Lys-NH$_2$), a vasopressin, a calcitonin or an insulin, more preferably GHRP or the RGD peptides cyclo(S,S)-N$^a$-acetyl-Cys-(N$^a$-methyl)Arg-Gly-Asp-Pen-NH$_2$ or cyclo(S,S)-(2-mercapto)benzoyl-(N$^a$-methyl)Arg-Gly-Asp-(2-mercapto)phenylamide.

In a prefered aspect, the present invention provides microemulsions comprising a peptide which may be orally administered and which will retain biological activity, thereby overcoming the disadvantages of earlier formulations in which the bioavailability of the peptide has been less than satisfactory. In particular, the present invention provides formulations which by their nature permit the preparation and administration of a peptide in sufficiently high concentration to allow not only convenient oral administration but also adequate bioavailability of the peptide.

For a water-soluble drug, the degree of incorporation into the (w/o) microemulsions of the present invention is limited by its solubility in the hydrophilic phase. The ionic strength and pH (within the range 3 to 10) may be adjusted to aid dissolution, without compromising the integrity of the microemulsion.

The aqueous hydrophilic phase suitably comprises water or an isotonic saline solution and may also include any pharmaceutically acceptable solvent which is non-miscible with the lipophilic phase.

In a preferred aspect, it has been found that in microemulsions of the present invention, the use of a mono- or polyhydroxyalcohol co-surfactant, such as ethanol, butanol or propylene glycol, as the major component of the hydrophilic phase may be avoided. This has the advantage of not only mitigating the stability and processing difficulties associated with the use of such but also reducing the concomitant stomach and duodenum irritation. Accordingly, the hydrophilic phase of microemulsions of the present invention may be essentially aqueous and comprise less than 10%, preferrably less than 5% and more preferrably less than 2% by weight of the phase of an alcohol.

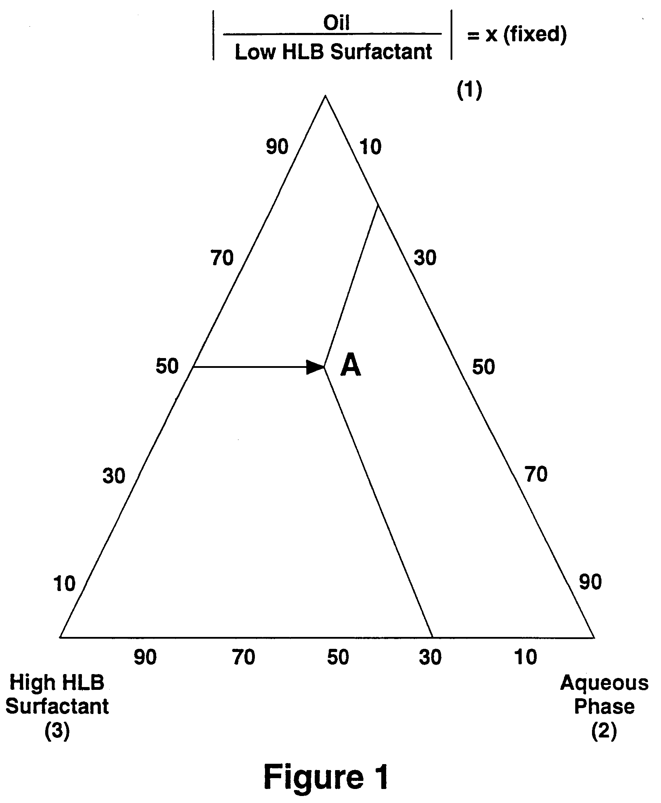

It will be readily appreciated by the skilled person that not all blends of a long-chain fatty acid triglyceride, low and high HLB surfactants and hydrophilic phase will yield stable, self-emulsifying microemulsions within the scope of the present invention. Appropriate ratios may, however, be readily determined by the skilled man with the aid of a phase diagram such as that illustrated in Fig. 1. As the system comprises four components *viz* a long-chain fatty acid triglyceride (oil), a low HLB surfactant, a high HLB surfactant and an aqueous/hydrophilic phase, a pseudo-ternary phase diagram is employed. In this, the ratio of two components such as the oil and the low HLB surfactant is kept constant so that there are only three variables, each of which can then be represented by one side of the triangle.

Thus, in Fig. 1, (1) represents the mixture of the oil and the low HLB surfactant, at a fixed ratio X, (2) the hydrophilic (aqueous) phase and (3) the high HLB surfactant. By way of example, the point "A" represents a mixture having 50% oil plus low HLB surfactant, 20% aqueous phase and 30% high HLB surfactant.

The regions of the phase diagram in which microemulsion according to the present invention exist may be determined by titrating a mixture of the oil and low HLB surfactant (in a fixed ratio) against the high HLB surfactant and the hydrophilic phase, noting points of phase separation, turbidity and transparency. Clear, transparent formulations are indicative of the formation of a stable microemulsion. Liquid and gel formulations may be obtained at room temperature according to the specific nature of the components employed.

Once stable transparent systems are obtained, simple tests, such as dye solubilization, dispersibility in water and conductivity measurements may be used to determine whether the microemulsion is an (o/w)- or a (w/o)-type. A water-soluble dye will disperse in an (o/w) microemulsion whilst it will remain in its original form in a (w/o) microemulsion. Likewise, (o/w) microemulsions are generally dispersible in water whereas (w/o) microemulsions are generally not. In addition, (o/w) microemulsions conduct electricity whereas (w/o) do not. The isotropic nature of the system may be confirmed by examination thereof under polarised light. The microemulsions being micellar in nature are isotropic and therefore non-birefringent when examined under polarised light.

From this phase diagram, appropriate percentages may then be read off. The process may then be repeated for other ratios of oil to low HLB surfactant so that an overall picture may be obtained.

EP 0 597 029 B1

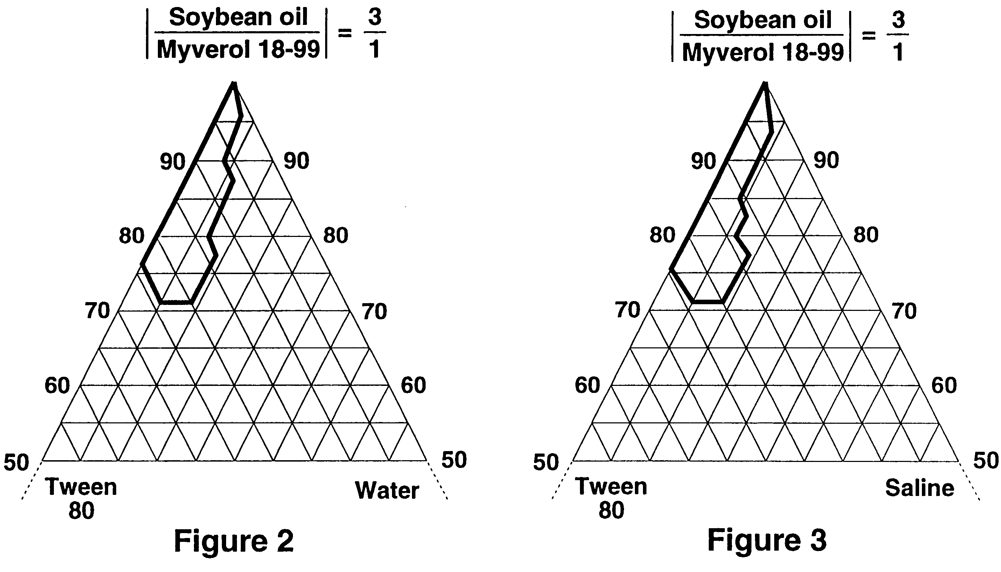

A representative pseudo-ternary phase diagram for systems containing a long-chain fatty acid triglyceride (soybean oil) and a low HLB surfactant (MYVEROL 18-99) (ratio 3:1), a high HLB surfactant (TWEEN 80) and water as the hydrophilic (aqueous) phase is shown in Figure 2. The mixture of oil plus the low HLB surfactant is indicated as component (1), water as component (2) and the high HLB surfactant as compontent (3). The phase diagram is a "partial" phase diagram as the study was limited to those relative amounts of the various components in which microemulsions were expected to be found. This system produces a range of clear, transparent microemulsions (the microemulsion existence field) which are shown in the phase diagram as the shaded area. The microemulsion existence field was essentially unchanged when water was replaced by saline, as shown in Fig. 3.

In general, stable clear, transparent liquid microemulsions were obtained when the oil plus low HLB surfactant was present in the range from about 70 to about 95% , the high HLB surfactant from about 5 to about 25% and the water less than 5% (w/w) of the microemulsion.

By this process of constructing a representative range of phase diagrams, it has been possible to determine appropriate quantities of the various components which will lead to stable, self-emulsifying microemulsions falling within the present invention.

Suitably, the long-chain fatty acid triglyceride and the low HLB surfactant together comprise from about 70 to about 95%, preferably about 85 to about 95%, (w/w) of the microemulsion. The long-chain fatty acid triglyceride and the low HLB surfactant may be combined and mixed at various ratios. Useful (w/o) microemulsions may be obtained when the ratio of long-chain fatty acid triglyceride to low HLB surfactant is in the range of about 4:1 to about 2:1, preferably about 4:1 to about 3:1.

Suitably, the high HLB surfactant is present in the range of about 5 to about 25%, preferably about 7.5 to about 15% (w/w) of the microemulsion.

Suitably, the hydrophilic phase comprises from just greater than 0 to about 10%, preferably from about 0.1 to about 5%, more preferably from about 2 to about 5% (w/w) of the microemulsion.

It will be readily appreciated by the skilled person that, in general, an increase in the relative amount of high HLB surfactant will have to be matched by an increase in the relative amount of hydrophilic phase.

In preferred microemulsions of the present invention, the lipophilic phase comprises from about 70 to 95%, suitably 85 to 95%, the high HLB surfactant from about 5 to 25%, suitably 7.5 to 15% and the hydrophilic phase less than 10%, suitably from 0.1 to 5% (w/w) of the microemulsion.

Within such microemulsions, the ratio of long-chain fatty acid triglyceride to low HLB surfactant is between 4:1 and 2:1, suitably between 4:1 and 3:1.

The microemulsions of the present invention are substantially non-opaque, that is they are transparent or opalescent when viewed by optical microscopic means. In their undisturbed state, they are optically isotropic (non-birefringent) when examined under polarized light. They generally exhibit excellent stability at low and ambient temperatures, without phase separation, clouding or precipitation, even over prolonged periods of time. The formulations may be stored in a stable form at various temperatures, such as at 4°C, ambient temperature, 37°C and 50°C, preferably at 4°C or ambient temperatures. Peptide-containing microemulsions of the present invention exhibit a similar stability (shelf life) profile to that of the corresponding peptide-free microemulsions. Stable (w/o) microemulsions may be formed when the pH of the aqueous phase varies from a pH of approximately 3 to about 10, a property that can be beneficial for drugs exhibiting higher solubility at low or high pH. Microemulsions with a relatively higher amount of a high HLB surfactant such as TWEEN 80 tend to be more viscous due to the greater viscosity of this material.

Preferably, the diameter of droplets or particles of microemulsions of the present invention, measured, for instance, as the number-average diameter by laser light scattering techniques, is less than 150 nm, more preferably less than 100 nm, yet more preferably less than 50 nm and most preferably in the range 5 to 35 nm.

The various phases may optionally contain further ingredients, such as, but not limited to:

(i) lipids, such as phospholipids, in particular lecithins, such as soya bean lecithins, egg lecithin or egg phosphatide, cholesterol or oleic acid;
(ii) antioxidants such as n-propyl gallate, butylated hydroxyanisole (BHA) and mixed isomers thereof, d-a-tocopherol and mixed isomers thereof, ascorbic acid, propylparaben, methylparaben and citric acid (monohydrate);
(iii) bile salts, for instance as their alkali metal salts, such as sodium taurocholate;
(iv) stabilizers, such as hydroxypropyl cellulose;
(v) antimicrobials, such as benzoic acid (sodium salt);
(vi) dioctylsuccinate, di-octylsodium sulfosuccinate or sodium lauryl sulfate; and
(vii) protease inhibitors such as aprotinin.

The microemulsions of the present invention form spontaneously or substantially spontaneously when their components are brought into contact, that is without the application of substantial energy supply, for instance in the absence of high shear energy such as imparted by homogenization and/or microfluidization or other mechanical agitation. Accordingly the microemulsions may be readily prepared by the simple process of admixing appropriate quantities, with

6

gentle hand mixing or stirring, if necessary, to ensure thorough mixing. Preferably, the drug is dissolved in the hydrophilic phase, either directly or by dilution of a stock solution thereof and this may then be added to a pre-mixed combination of the oil and the low HLB surfactant with mixing, followed by the high HLB surfactant or *vice versa*. Alternatively, a drug-free microemulsion may be initially prepared by admixing the oil, low and high HLB surfactants and some (drug-free) hydrophilic phase to which may then be added drug dissolved in hydropbilic phase. If necessary, higher temperatures (40-60°C) may be used to assist the solubilization of all of the components during the preparation of the microemulsion. Formulation at ambient temperature is, however, preferred for thermolabile active ingredients, such as peptides.

Microemulsions of the present invention are pharmaceutical compositions which comprise a therapeutic agent and are therefore intended for use in therapy, for administration to animals, including man.

It will be recognized by one of skill in the art that the amount of drug required for therapeutic effect on administration will, of course, vary with the agent chosen, the nature and severity of the condition and the animal undergoing treatment, and is ultimately at the discretion of the physician.

Furthermore, the optimal quantity and spacing of individual dosages of a drug will be determined by the nature and extent of the condition being treated, the form, route and site of administration, the particular patient being treated and that such optima can be determined by conventional techniques. It will also be appreciated that the optimal course of treatment, that is, the number of doses given, can be ascertained by those skilled in the art using conventional course of treatment determination tests.

The present invention also provides for the use of a long-chain fatty acid triglyceride, a low HLB surfactant, a high HLB surfactant, a therapeutic agent and a hydrophilic phase as hereinbefore defined in the manufacture of a medicament.

Microemulsions of the present invention may be used for oral, topical, rectal, intra-vaginal or other forms of systemic administration and accordingly will be presented in forms suitable for such. Thus for instance, microemulsions intended for oral administration may be presented in soft gelatin capsules whilst the viscosity characteristics of some of the microemulsions make them suitable for direct topical application. Compositions adapted for oral or topical administration are especially prefered.

The microemulsions of the present invention without a drug are novel and useful as precursors to drug-containing microemulsions. Accordingly, in a further aspect, the present invention provides a stable, self-emulsifying water-in-oil microemulsion comprising:

(a) a lipophilic phase having a long-chain fatty acid triglyceride and a low HLB surfactant; (b) a high HLB surfactant; and (c) an aqueous hydrophilic phase in which each of (a), (b) and (c) are as hereinbefore defined and pharmaceutically acceptable.

The invention will now be illustrated by, but not limited to, the following descriptions (drug-free microemulsions) and examples (drug-containing microemulsions) and biological examples.

**DESCRIPTIONS**

**Description 1 - Phase Diagrams (partial) for Representative Microemulsions**

Representative pseudo-ternary partial phase diagrams (fig. 2 and 3) for the system soybean oil, MYVEROL 18-99 (ratio 3:1), TWEEN 80 and water or saline have already been described elsewhere. This study was extended to other systems in which the oil and the high HLB surfactant were kept constant whilst the low HLB surfactant, the ratio thereof to the oil and the aqueous phase were varied, as shown in the table 1:

Table 1

| Systems for phase diagrams | | | |
|---|---|---|---|
| Description no. (fig.) | Low HLB surfactant | Ratio oil to low HLB surfactant | Aqueous |
| 1a/(fig.2) | MYVEROL 18-99 | 3:1 | deionised water |
| 1b/(fig.3) | MYVEROL 18-99 | 3:1 | saline |
| 1c/(fig.4) | ARLACEL 186 | 3:1 | deionised water |
| 1d/(fig.5) | ARLACEL 83 | 3:1 | deionised water |
| 1e/(fig.6) | ARLACEL 80 | 3:1 | deionised water |
| 1f/(fig.7) | ARLACEL 80 | 4:1 | deionised water |

The various components were weighed out individually and then mixed at a temperature between 40 and 50°C. The relative order of addition would appear to be unimportant. For components such as MYVEROL 18-99 which has a melting point of about 42°C and is therefore a solid at room temperature, it was useful to premelt at an appropriate temperature, prior to addition to assist in complete mixing and solubilisation. Heating at about 50°C was also usefully be employed during subsequent mixing. Further equilibriation of the microemulsion for a 24 h period was found to improve the stability of some microemulsions.

The regions of the phase diagram in which microemulsions according to the present invention exist were determined by titrating a mixture of the oil and low HLB surfactant (in a fixed ratio) against the high HLB surfactant and the aqueous phase, noting points of phase separation, turbidity and transparency. Clear, transparent formulations are indicative of the formation of a stable microemulsion.

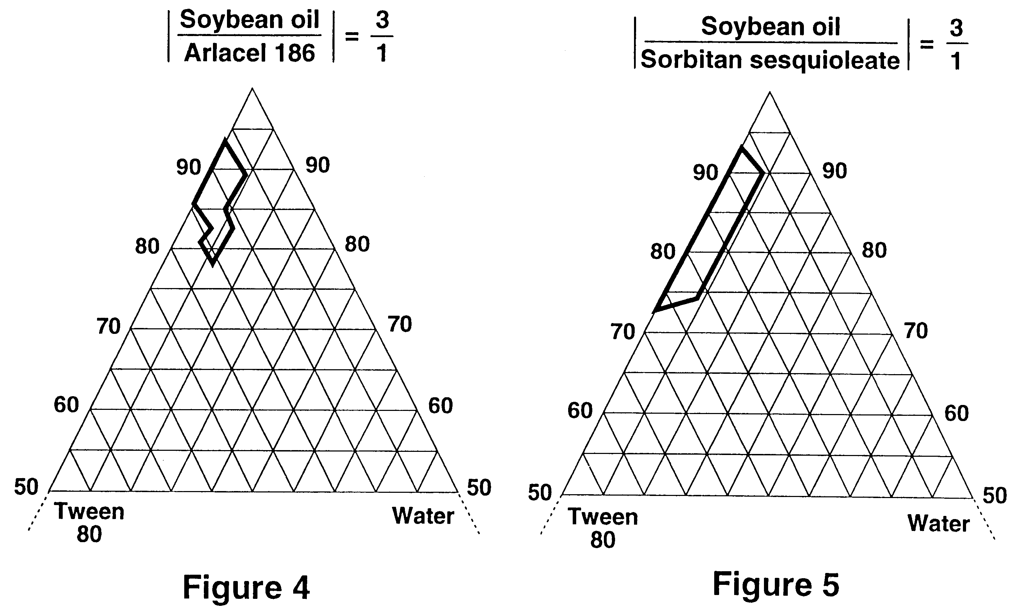

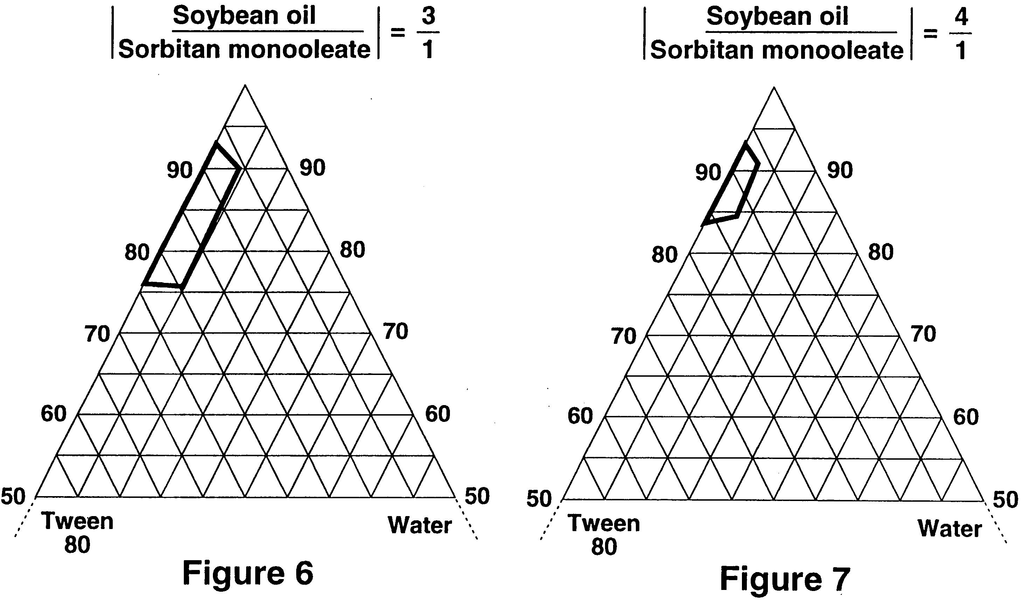

Representative partial phase diagrams with emphasis on the microemulsion-existence field are shown as figure 2 and 3 for the MYVEROL 18-99 system. Similar phase diagrams were also obtained for the other systems containing other low HLB surfactant and these are shown as Figures 4 to 7. Clear, transparent microemulsions were obtained in the shaded areas on the phase diagrams (the microemulsion existence field). When examined under polarised light, non-birefringent behaviour was observed. These microemulsions were also found to have extremely low electrical conductance. In general, replacing water by saline had no effect on the microemulsion existence field, other than small changes in the boundary region. Changing the ratio of oil to low HLB surfactant from 3:1 to 4:1 resulted in the formation of a narrower field, due to the reduced amount of low HLB surfactant. The microemulsions were however otherwise similar to those obtained in the "3:1" systems.

The ranges of the components for selected systems in which microemulsion fields were observed is given in table 2:

Table 2

| Description no. | lipophilic phase % | High HLB surfactant % | hydrophilic phase % |
|---|---|---|---|
| 1a | 70 to 90 | 5 to 25 | <10 |
| 1b | 70 to 90 | 5 to 25 | <10 |
| 1c | 80 to 95 | 5 to 15 | <10 |

These phase diagrams show that microemulsions within the scope of the present invention are obtained for ratios of long-chain fatty acid triglyceride to low HLB surfactant ranging from 4:1 to 3:1. Clear and transparent liquid microemulsions similar to those described above were also obtained at oil to low HLB surfactant ratios of 2:1.

Various physical characteristics of the microemulsions hereinbefore described are given in the table 3. All the microemulsions had oil plus low HLB surfactant (3:1, 87%), high HLB surfactant (10%) and aqueous (3%).

Table 3

| Physical Properties of Microemulsions | | | |
|---|---|---|---|
| Description no. | Viscosity[a] (centiPoise) | Refractive index[a] | Conductance[b] (micromhos/cm) |
| 2 | 110.7 | 1.471 | 0.131 |
| 1e | 129.3 | 1.471 | 0.130 |
| 1g | 145.3 | 1.472 | 0.130 |
| 1d | 125.1 | 1.469 | 0.177 |
| Oleic Acid[c] | 23.4(25.6) | 1.4582(1.4583) | ND |
| Deionized Water | ND | ND | 2.67 |
| Saline | ND | ND | 13400 |

[a] aqueous phase = water
[b] aqueous phase = saline
[c] the viscosity and refractive index values shown in parentheses are from the CRC Handbook of Chemistry and Physics, 60th Edition.

Measurement of particle diameter by laser light scattering of the representatitive microemulsions showed that they were mostly in the range 5 to 30nm and had a high degree of homogeneity (polydispersity in the range 0.05 to 0.20). This was not significantly affected by the inclusion of a peptide (as in example 1a, at 1 or 3mg/g formulation).

Microemulsions comprising glycerol monooleate (MYVEROL 18-99, mp about 42°C) as the low HLB surfactant formed gels at low temperature (4°C) which upon reequilibriation at room temperature or 37°C returned to clear, transparent microemulsions. After several weeks storage at room temperature, some precipitation and solidification was observed. This was however reversed by warming up to 37 or 50°C. At these temperatures, the microemulsions were stable for over one month, without any precipitation and/or phase seperation. In corresponding microemulsions still containing glycerol monooleate but using the product MYVEROL 18-99 rather than ARLACEL 186, similar one month storage stability was observed at 37 and 50°C. At 4°C, a gel was formed but this had much better long-term stability after returning to room temperature. Excellent storage characteristics were also observed for microemulsions comprising either sorbitan monooleate (ARLACEL 80) or sorbitan sesquioleate (ARLACEL 83) as the low HLB surfactant.

**Description 2 - (w/o) microemulsion**

A representative water-in-oil microemulsion was prepared on a 5g scale using the following:

| | |
|---|---|
| Soybean oil and MYVEROL 18-99 (ratio 3:1) | 87% |
| TWEEN 80 | 10 |
| Deionised water or saline | 3 |

MYVEROL 18-99 was premelted on a water bath before being added to soybean oil in a vial on a hot plate stirrer maintained at 50°C, with gentle stirring. This mixture was then added to a stirred mixture of TWEEN 80 and water or saline to form a clear transparent (w/o) microemulsion. Additional equilibriation at 50°C was found to improve stability. At low to ambient temperatures, the liquid formulation became a gel but this was reversed on warming to higher temperature.

**EXAMPLES**

For further studies on microemulsions incorporating a drug, an optimal formulation was selected from about the centre of the microemulsion field of the phase diagrams hereinbefore described. This formulation comprised soybean oil plus low HLB surfactant (87%), TWEEN 80 (10%) and a variable aqueous phase (3%).

These microemulsions were generally formulated by initially preparing the drug-containing hydrophilic phase, either by dissolving the appropriate amount of drug in the appropriate amount of water or, more preferably, using a stock solution which was then further diluted if so required, with vortex stirring if necessary to obtain complete dissolution. The hydrophilic phase containing the drug was then added to the appropriate amounts (by weight) of a mixture of the oil and the low HLB surfactant, to which was then added the high HLB surfactant, with gentle stirring (magnetic hot plate stirrer). Alternatively, the hydrophilic phase containing the drug was added to the high HLB surfactant and following upon complete mixing, this was added to the oil plus low HLB surfactant mixture. If necessary, the drug-containing microemulsion was then diluted with the corresponding drug-free microemulsion to adjust the concentration of the drug. Batches were routinely prepared on a 5 or 10 g scale. In addition larger scale (50 to 500g) batches were also prepared.

Following the standard procedure outlined above, the following drug-containing microemulsions were prepared, as shown in the table 4:

Table 4

| Examples of drug-containing microemulsions | | | |
|---|---|---|---|
| Example | Drug | Drug conc. mg/g form. | high HLB surfactant |
| 1a | RGD peptide[a] | 1-6 | MYVEROL 18-99 |
| 1b | RGD peptide[a] | 6 | ARLACEL 80 |
| 1c | RGD peptide[a] | 6 | ARLACEL83 |
| 1d | RGD peptide[a] | 6 | ARLACEL186 |
| 2 | GHRP[b] | 3.0 | MYVEROL 18-99 |
| 3 | vasopressin[c] | 0.06 | ARLACEL186 |
| 4a | calcitonin[d] | 0.02 | ARLACEL186 |
| 4b | calcitonin[d] | 0.09 | ARLACEL186 |
| 5a | insulin[e] | 0.15 (37 IU) | ARLACEL186 |
| 5b | insulin[e] | 0.38 (92 IU) | ARLACEL186 |

**Footnotes to table**

[a] cyclo(S,S)-(2-mercapto)benzoyl-($N^a$-methyl)-Arg-Gly-Asp-(2-mercapto)-phenylamide (MW of about 650), aq. = saline;

[b] His-D-Trp-Ala-Trp-D-Phe-Lys-$NH_2$ (MW of about 850), aq. = isotonic soln containing acetic acid and NaCl at pH 5.0;

[c] Val-Asp-Cys-Tyr-Phe-Gln-Asn-Cys-Pro-Arg-Gly-$NH_2$ (MW of about 1300) (ICN Biochemicals), aq. = saline;

[d] salmon calcitonin (contains 32 amino acids, MW of about 3500) (ICN Biochemicals), aq. = saline; and

[e] polypeptide with a MW of about 6000 (ICN Biochemicals), aq. = phosphate-buffered saline.

## BIOLOGICAL EXAMPLES

### Biological Example 1- Assessment of GI Irritation

Using standard methodology (Szabo *et al.*, J. Pharmacol. Methods, **13**, 59-66, 1985), a drug-free microemulsion comprising soybean oil and one of MYVEROL 18-99, ARLACEL 80, ARLACEL 83 or ARLACEL186 (ratio 3:1) (87%), TWEEN 80 (10%) and saline (3%) was assessed for its potential to cause GI irritation in rats. After oral dosing (3.3 ml/kg), the mucosal surfaces of both the stomach and duodenum were found to be free of any lesions, even under microscopic examination.

### Biological Example 2 - Demonstration of *in vivo* activity of RGD peptide delivered by a microemulsion

The *in vivo* activity of microemulsions of containing soybean oil and one of MYVEROL 18-99, ARLACEL 80, ARLACEL 83 or ARLACEL186 (ratio 3:1) (87%), TWEEN 80 (10%) and saline (3%) and the RGD petide of example 1a (6mg/g formulation) was demonstrated in a standard platelet aggregation assay using dogs (Samanen *et al.*, Med Chem., **34**, 3114-3125, 1991). Following oral dosing of the microemulsion containing the peptide (in a gelatin capsule) at 3 mg/kg (0.5 ml/kg microemulsion), inhibition was observed which was, in general, more pronounced and, in some cases, more sustained than that observed in a corresponding control experiment in which the RGD peptide was dosed as a saline solution.

**Claims**

1.   A pharmaceutically acceptable, stable, self-emulsifying water-in-oil microemulsion comprising:

    (a) a lipophilic phase having as an oil, a long-chain fatty acid triglyceride, and a low HLB surfactant;
    (b) a high HLB surfactant selected from a polyoxyethylene fatty acid ester, a polyoxyethylene-sorbitan fatty acid ester, a polyoxyethylene glycol long-chain alkyl ether or a polyoxyethylene glycol long-chain alkyl ester;
    (c) an aqueous hydrophilic phase; and
    (d) a water-soluble therapeutic agent.

2.   A microemulsion as claimed in claim 1 in which the major fatty acid components of the long-chain fatty acid triglyceride are $C_{18}$-saturated, monounsaturated or polyunsaturated fatty acids.

3.   A microemulsion as claimed in claim 1 or 2 in which the low HLB surfactant is (a) a sorbitan long-chain fatty acid ester; or (b) a long-chain fatty acid monoglyceride optionally comprising up to 10% (w/w) of a long-chain fatty acid diglyceride and/or a small amount by weight of a free long-chain fatty acid.

4.   A microemulsion as claimed in claim 3 in which the long-chain fatty acid is a $C_{18}$-saturated, monounsaturated or polyunsaturated fatty acid.

5.   A microemulsion as claimed in claim 1 in which the long-chain fatty acid triglyceride is soybean oil or safflower oil and the low HLB surfactant is glycerol monooleate, sorbitan monooleate or sorbitan sesquioleate.

6.   A microemulsion as claimed in any one of claims 1 to 5 in which the therapeutic agent is a peptide.

7.   A microemulsion as claimed in claim 6 in which the peptide is a fibrinogen receptor antagonist peptide (an RGD peptide), GHRP (His-D-Trp-Ala-Trp-D-Phe-Lys-$NH_2$), a vasopressin, a calcitonin or an insulin.

8.   A microemulsion as claimed in any one of claims 1 to 7 in which the the hydrophilic phase is essentially aqueous and comprises less than 10% by weight of the total microemulsion.

9.   A microemulsion as claimed in any one of claims 1 to 8 in which the lipophilic phase comprises from 70 to 95%, the high HLB surfactant from 5 to 25% and the hydrophilic phase less than 10% and in which the ratio of long-chain fatty acid triglyceride to low HLB surfactant is between 4:1 and 2:1.

10.   A microemulsion as claimed in claim 10 in which the lipophilic phase comprises from 85 to 95%, the high HLB surfactant from 7.5 to 15% and the hydrophilic phase from 0.1 to 5% (w/w) of the microemulsion.

11.   A self-emulsifying water-in-oil (w/o) microemulsion optionally comprising a water-soluble therapeutic agent in which the relative proportions of the following components:

    (1) a lipophilic phase comprising a long-chain fatty acid triglyceride or a blend of long-chain fatty acid triglycerides and a low HLB surfactant or a blend of low HLB surfactants;
    (2) a high HLB surfactant selected from a polyoxyethylene fatty acid ester, a polyoxyethylene-sorbitan fatty acid ester, a polyoxyethylene glycol long-chain alkyl ether or a polyoxyethylene glycol long-chain alkyl ester; and
    (3) an aqueous phase
    lie within any one of the indicated regions of

        a) figure 2 related to $\frac{\text{soybean oil}}{\text{myverol 18-99}} = \frac{3}{1}$, Tween 80 and water,
        b) figure 3 related to $\frac{\text{soyabean oil}}{\text{myverol 18-99}} = \frac{3}{1}$, Tween 80 and saline,
        c) figure 4 related to $\frac{\text{soyabean oil}}{\text{arlacel 186}} = \frac{3}{1}$, Tween 80 and water,
        d) figure 5 related to $\frac{\text{Soyabean oil}}{\text{Sorbitan sesquioleate}} = \frac{3}{1}$, Tween 80 and water,
        e) figure 6 related to $\frac{\text{Soyabean oil}}{\text{Sorbitan monoaleate}} = \frac{3}{1}$, Tween 80 and water and

f) figure 7 related to $\frac{Soyabean\ oil}{Sorbitan\ monoaleate} = \frac{4}{1}$, Tween 80 and water.

12. A microemulsion as claimed in any one of claims 1 to 12 adapted for oral delivery.

13. A microemulsion as claimed in any one of claims 1 to 12 adapted for topical application.

14. The use of a microemulsion as defined in anyone of claims 1 to 12 comprising a long-chain fatty acid triglyceride, a low HLB surfactant, a high HLB surfactant, a therapeutic agent and a hydrophilic phase in the manufacture of a medicament.

**Patentansprüche**

1. Pharmazeutisch verträgliche, stabile, selbstemulgierende Wasser-in-Öl-Mikroemulsion, umfassend:

   (a) eine lipophile Phase, die, als ein Öl, ein Langkettenfettsäure-Triglycerid, und ein grenzflächenaktives Mittel mit niedrigem HLB-Wert besitzt;
   (b) ein grenzflächenaktives Mittel mit hohem HLB-Wert, ausgewählt aus einem Polyoxyethylen-Fettsäureester, einem Polyoxyethylen-Sorbitan-Fettsäureester, einem Polyoxyethylenglycol-Langkettenalkyl-Ether oder einem Polyoxyethylenglycol-Langkettenalkyl-Ester;
   (c) eine wäßrige hydrophile Phase und
   (d) einen wasserlöslichen therapeutischen Wirkstoff.

2. Mikroemulsion nach Anspruch 1, wobei die Hauptfettsäurekomponenten des Langkettenfettsäure-Triglycerides $C_{18}$-gesättigte, einfach ungesättigte oder mehrfach ungesättigte Fettsäuren sind.

3. Mikroemulsion nach einem der Ansprüche 1 oder 2, wobei das grenzflächenaktive Mittel mit niedrigem HLB-Wert (a) ein Sorbitan-Langkettenfettsäure-Ester oder (b) ein Langkettenfettsäure-Monoglycerid ist, das gegebenenfalls bis zu 10% (w/w) eines Langkettenfettsäure-Diglycerides und/oder eine kleine Gewichtsmenge einer freien langkettigen Fettsäure umfaßt.

4. Mikroemulsion nach Anspruch 3, wobei die langkettige Fettsäure eine $C_{18}$-gesättigte, einfach ungesättigte oder mehrfach ungesättigte Fettsäure ist.

5. Mikroemulsion nach Anspruch 1, wobei das Langkettenfettsäure-Triglycerid Sojabohnenöl oder Saffloröl ist und das grenzflächenaktive Mittel mit niedrigem HLB-Wert Glycerinmonooleat, Sorbitan-Monooleat oder Sorbitan-Sesquioleat ist.

6. Mikroemulsion nach einem der Ansprüche 1 bis 5, wobei der therapeutische Wirkstoff ein Peptid ist.

7. Mikroemulsion nach Anspruch 6, wobei das Peptid ein Fibrinogenrezeptorantagonist-Peptid (ein RGD-Peptid), GHRP (His-D-Trp-Ala-Trp-D-Phe-Lys-NH$_2$), ein Vasopressin, ein Calcitonin oder ein Insulin ist.

8. Mikroemulsion nach einem der Ansprüche 1 bis 7, wobei die hydrophile Phase im wesentlichen wäßrig ist und weniger als 10 Gew.-% der Gesamtmikroemulsion umfaßt.

9. Mikroemulsion nach einem der Ansprüche 1 bis 8, wobei die lipophile Phase von 70 bis 95%, das grenzflächenaktive Mittel mit hohem HLB-Wert von 5 bis 25% und die hydrophile Phase weniger als 10% umfaßt und wobei das Verhältnis von Langkettenfettsäure-Triglycerid zu grenzflächenaktivem Mittel mit niedrigem HLB-Wert zwischen 4:1 und 2:1 liegt.

10. Mikroemulsion nach Anspruch 9, wobei die lipophile Phase von 85 bis 95%, das grenzflächenaktive Mittel mit hohem HLB-Wert von 7,5 bis 15% und die hydrophile Phase von 0,1 bis 5% (w/w) der Mikroemulsion umfaßt.

11. Selbstemulgierende Wasser-in-Öl- (w/o-) Mikroemulsion, die gegebenenfalls einen wasserlöslichen therapeutischen Wirkstoff umfaßt, wobei die relativen Anteile der nachstehenden Komponenten:

    (1) eine lipophile Phase, umfassend ein Langkettenfettsäure-Triglycerid oder ein Gemisch aus Langkettenfettsäure-Triglyceriden und einem grenzflächenaktiven Mittel mit niedrigem HLB-Wert oder einem Gemisch aus grenzflächenaktiven Mitteln mit niedrigem HLB-Wert;

(2) ein grenzflächenaktives Mittel mit hohem HLB-Wert, ausgewählt aus einem Polyoxyethylen-Fettsäureester, einem Polyoxyethylen-Sorbitan-Fettsäureester, einem Polyoxyethylenglycol-Langkettenalkyl-Ether oder einem Polyoxyethylenglycol-Langkettenalkyl-Ester und

(3) eine wäßrige Phase

innerhalb eines der angegebenen Bereiche von

    a) Figur 2 bezüglich Sojabohnenöl/Myverol 18-99 = 3/1, Tween 80 und Wasser,
    b) Figur 3 bezüglich Sojabohnenöl/Myverol 18-99 = 3/1, Tween 80 und Kochsalzlösung,
    c) Figur 4 bezüglich Sojabohnenöl/Arlacel 186 = 3/1, Tween 80 und Wasser,
    d) Figur 5 bezüglich Sojabohnenöl/Sorbitan-Sesquioleat = 3/1, Tween 80 und Wasser,
    e) Figur 6 bezüglich Sojabohnenöl/Sorbitan-Monooleat = 3/1, Tween 80 und Wasser und
    f) Figur 7 bezüglich Sojabohnenöl/Sorbitan-Monooleat = 4/1, Tween 80 und Wasser liegen.

**12.** Mikroemulsion nach einem der Ansprüche 1 bis 12, die an die orale Einnahme angepaßt ist.

**13.** Mikroemulsion nach einem der Ansprüche 1 bis 12, die an die topische Anwendung angepaßt ist.

**14.** Verwendung einer Mikroemulsion nach einem der Ansprüche 1 bis 12, umfassend ein Langkettenfettsäure-Triglycerid, ein grenzflächenaktives Mittel mit niedrigem HLB-Wert, ein grenzflächenaktives Mittel mit hohem HLB-Wert, einen therapeutischen Wirkstoff und eine hydrophile Phase, zur Herstellung eines Medikamentes.

## Revendications

**1.** Micro-émulsion eau-dans-huile auto-émulsionnable, stable, pharmaceutiquement acceptable, comprenant :

(a) une phase lipophile comprenant comme huile un triglycéride d'acide gras à chaîne longue, et un surfactant à faible valeur d'équilibre hydrophile-lipophile (EHL) ;
(b) un surfactant à valeur de EHL élevée, choisi entre un ester d'acide gras de polyoxyéthylène, un ester d'acide gras de polyoxyéthylène-sorbitanne, un éther alkylique à chaîne longue de polyoxyéthylène-glycol ou un ester alkylique à chaîne longue de polyoxyéthylène-glycol ;
(c) une phase hydrophile aqueuse ; et
(d) un agent thérapeutique hydrosoluble.

**2.** Micro-émulsion suivant la revendication 1, dans laquelle les acides gras présents comme constituants principaux du triglycéride d'acides gras à chaîne longue sont des acides gras en $C_{18}$ saturés, mono-insaturés ou polyinsaturés.

**3.** Micro-émulsion suivant la revendication 1 ou 2, dans laquelle le surfactant à faible valeur de EHL est (a) un ester de sorbitanne et d'acide gras à chaîne longue ; ou (b) un monoglycéride d'acide gras à chaîne longue comprenant facultativement jusqu'à 10 % (en poids/poids) d'un diglycéride d'acide gras à chaîne longue et/ou une petite quantité en poids d'un acide gras libre à chaîne longue.

**4.** Micro-émulsion suivant la revendication 3, dans laquelle l'acide gras à chaîne longue est un acide gras en $C_{18}$ saturé, mono-insaturé ou polyinsaturé.

**5.** Micro-émulsion suivant la revendication 1, dans laquelle le triglycéride d'acide gras à chaîne longue est l'huile de soja ou l'huile de carthame et le surfactant à faible valeur de EHL est le mono-oléate de glycérol, le mono-oléate de sorbitanne ou le sesquioléate de sorbitanne.

**6.** Micro-émulsion suivant l'une quelconque des revendications 1 à 5, dans laquelle l'agent thérapeutique est un peptide.

**7.** Micro-émulsion suivant la revendication 6, dans laquelle le peptide est un peptide antagoniste des récepteurs du fibrinogène (un peptide RGD), le GHRP (His-D-Trp-Ala-Trp-D-Phe-Lys-$NH_2$), une vasopressine, une calcitonine ou une insuline.

**8.** Micro-émulsion suivant l'une quelconque des revendications 1 à 7, dans laquelle la phase hydrophile est essentiellement aqueuse et représente moins de 10 % en poids de la micro-émulsion totale.

9. Micro-émulsion suivant l'une quelconque des revendications 1 à 8, dans laquelle la phase lipophile représente 70 à 95 %, le surfactant à valeur élevée de EHL représente 5 à 25 % et la phase hydrophile représente moins de 10 %, et dans laquelle le rapport du triglycéride d'acide gras à chaîne longue au surfactant à faible valeur de EHL est compris dans l'intervalle de 4:1 à 2:1.

10. Micro-émulsion suivant la revendication 9 , dans laquelle la phase lipophile représente 85 à 95 %, le surfactant à valeur de EHL élevée représente 7,5 à 15 % et la phase hydrophile représente 0,1 à 5 % (en poids/poids) de la micro-émulsion.

11. Micro-émulsion eau-dans-huile (E/H) autoémulsionnable, comprenant facultativement un agent thérapeutique hydrosoluble, dans laquelle les proportions relatives des constituants suivants :

    (1) une phase lipophile comprenant un triglycéride d'acide gras à chaîne longue ou un mélange de triglycérides d'acides gras à chaîne longue, et un surfactant à faible valeur de EHL ou un mélange de surfactants à faibles valeurs de EHL ;
    (2) un surfactant à valeur de EHL élevée choisi entre un ester d'acide gras de polyoxyéthylène, un ester d'acide gras de polyoxyéthylène-sorbitanne, un éther alkylique à chaîne longue de polyoxyéthylène-glycol ou un ester alkylique à chaîne longue de polyoxyéthylène-glycol ; et
    (3) une phase aqueuse se situent dans l'une quelconque des régions indiquées sur

      (a) la figure 2 concernant le rapport $\frac{\text{huile de soja}}{\text{Myverol 18-99}} = \frac{3}{1}$, le Tween 80 et l'eau,

      (b) la figure 3 concernant le rapport $\frac{\text{huile de soja}}{\text{Myverol 18-99}} = \frac{3}{1}$, le Tween 80 et une solution de sel,

      (c) la figure 4 concernant le rapport $\frac{\text{huile de soja}}{\text{Arlacel 186}} = \frac{3}{1}$, le Tween 80 et l'eau,

      (d) la figure 5 concernant le rapport $\frac{\text{huile de soja}}{\text{sesquioléate de sorbitanne}} = \frac{3}{1}$, le Tween 80 et l'eau,

      (e) la figure 6 concernant le rapport $\frac{\text{huile de soja}}{\text{mono} - \text{oléate de de sorbitanne}} = \frac{3}{1}$, le Tween 80 et l'eau, et

      (f) la figure 7 concernant le rapport $\frac{\text{huile de soja}}{\text{mono} - \text{oléate de de sorbitanne}} = \frac{4}{1}$, le Tween 80 et l'eau.

12. Micro-émulsion suivant l'une quelconque des revendications 1 à 11, apte à l'administration orale.

13. Micro-émulsion suivant l'une quelconque des revendications 1 à 12, apte à l'application topique.

14. Utilisation d'une micro-émulsion répondant à la définition suivant l'une quelconque des revendications 1 à 12, comprenant un triglycéride d'acide gras à chaîne longue, un surfactant à faible valeur de EHL, un surfactant à valeur de EHL élevée, un agent thérapeutique et une phase hydrophile dans la production d'un médicament.

$$\left| \frac{\text{Oil}}{\text{Low HLB Surfactant}} \right| = x \text{ (fixed)}$$

(1)

**Figure 1**

EP 0 597 029 B1

$$\left| \frac{\text{Soybean oil}}{\text{Myverol 18-99}} \right| = \frac{3}{1}$$

**Figure 2**

$$\left| \frac{\text{Soybean oil}}{\text{Myverol 18-99}} \right| = \frac{3}{1}$$

**Figure 3**

$$\left|\frac{\text{Soybean oil}}{\text{Arlacel 186}}\right| = \frac{3}{1}$$

**Figure 4**

$$\left|\frac{\text{Soybean oil}}{\text{Sorbitan sesquioleate}}\right| = \frac{3}{1}$$

**Figure 5**

EP 0 597 029 B1

EP 0 597 029 B1

$$\left|\frac{\text{Soybean oil}}{\text{Sorbitan monooleate}}\right| = \frac{3}{1}$$

**Figure 6**

$$\left|\frac{\text{Soybean oil}}{\text{Sorbitan monooleate}}\right| = \frac{4}{1}$$

**Figure 7**